(19) Europäisches Patentamt
European Patent Office
Office européen des brevets

(11) Veröffentlichungsnummer: **0 101 412**
**B1**

(12) # EUROPÄISCHE PATENTSCHRIFT

(45) Veröffentlichungstag der Patentschrift: **29.03.89**

(51) Int. Cl.⁴: **A 61 K 31/425** // (A61K31/425, 31:415)

(21) Anmeldenummer: **83810351.3**

(22) Anmeldetag: **08.08.83**

(54) **Anthelmintika.**

(30) Priorität: **13.08.82 CH 4866/82**

(43) Veröffentlichungstag der Anmeldung: **22.02.84 Patentblatt 84/8**

(45) Bekanntmachung des Hinweises auf die Patenterteilung: **29.03.89 Patentblatt 89/13**

(84) Benannte Vertragsstaaten: **BE CH DE FR IT LI NL SE**

(56) Entgegenhaltungen: **DE-A-2 815 621**

(73) Patentinhaber: **CIBA- GEIGY AG, Klybeckstrasse 141, CH- 4002 Basel (CH)**

(72) Erfinder: **Boray, Joseph C., Dr., 21 Bogota Avenue, Neutral Bay, N.S.W. 2089 (AU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die vorliegende Erfindung betrifft neue synergistische anthelmintische Mittel, die als aktive Komponente eine Wirkstoffkombination enthalten, sowie deren Verwendung zur Herstellung eines anthelminthischen Mittels zur Anwendung in der Bekämpfung von Helminthen, insbesondere von Trematoden und Nematoden, in Haus- und Nutztieren.

Die den erfindungsgemässen Mitteln zugrundeliegenden Wirkstoffkombinationen weisen eine unerwartete synergistische Wirksamkeit auf, welche die additive Wirkung der kombinierten Einzelwirkstoffe in sehr vorteilhafter Weise übertrifft. Die erfindungsgemässen Wirkstoffkombinationen bestehen aus folgenden Einzelwirkstoffen:

Eine Verbindung der Formel I

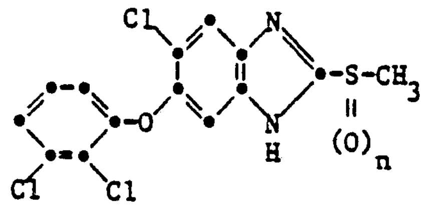

(I),

in welcher n gleich 0 oder 1 bedeutet, und der Verbindung der Formel II

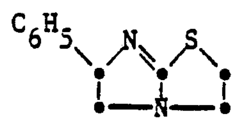

(II)

einschliesslich der unter die Formel II fallenden Isomeren sowie ihrer Säureadditionssalze.

Die als Wirkstoffkomponenten der erfindungsgemässen Mittel vorstehend beschriebenen Verbindungen sind individuell als anthelminthisch wirksam bekannt. Verbindungen der Formel I sind beispielsweise in der deutschen Offenlegungsschrift DE-A-2 815 621 und die Verbindung der Formel II in den US-Patenten US-A-3 274 209, 3 565 907 und 3 579 530, in der französischen Patentschrift FR-A-1 544 972, in Tetrahedron Letters 1967, 1467 und in Am. Journ. Vet. Res. 32, (1971) 545 beschrieben.

Die kombination von verschiedenen chemischen Verbindungen zur Erzeugung synergistischer Wirkungsverstärkung für die Bekämpfung von Schädlingen erlangt durch die damit gegebene Möglichkeit, der immer häufiger in Erscheinung tretenden Resistenz der Parasiten gegenüber Einzelverbindungen wirksam entgegenzutreten, in zunehmendem Masse an Bedeutung. Eine wegen des Auftretens von Resistenzerscheinungen bei den Parasiten notwendig werdende Erhöhung der zum Einsatz gelangenden Wirkstoffmengen kann somit weitgehend vermieden werden. Die sich daraus ergebende Einsparung an Substanzmengen bringt sowohl wirtschaftliche als auch durch Verminderung der Umweltbelastung bedeutsame ökologische Vorteile mit sich. Für die Verabreichung von Anthelminthika an Nutztieren kann das die Beibehaltung der tolerierbaren Applikationsdosis oder sogar deren Senkung bedeuten, womit gleichzeitig die Intoxikation der behandelten Tiere vermieden wird, so dass ihre Nutzleistung während der Therapie nicht beeinträchtigt wird.

Wurmerkrankungen von Haus- und Nutztieren sind bekanntermassen weit verbreitet und führen zur Hemmung des Wachstums und zu verminderter Nutzleistung der erkrankten Tiere, in vielen Fällen sogar zum Tode. Eine besonders gefährliche Wurmkrankheit stellt die Fasciolose dar, die durch parasitierende Leberegel (beispielsweise Fasciola hepatica und Fasciola gigantica) hervorgerufen wird und vor allem bei Wiederkäuern, wie Schafen und Rindern, auftritt. Die durch diese Wurmkrankheit hervorgerufenen Schäden können vor allem bei epidemieartigem Auftreten des Wurmbefalls in Viehherden ein beträchtliches Ausmass annehmen. Bekämpfung und Vorbeugung der Fasciolose gelten deshalb als vordringliche Aufgabe, um derartige vor allem volkswirtschaftlich ins Gewicht fallende Schäden in der tierischen Produktion zu vermeiden. Trotz zahlreicher bekannter Präparate auf dem Gebiet der Helminthenbekämpfung ist es bisher nicht gelungen, Wirkstoffe zu entwickeln, die neben guten allgemeinen anthelminthischen Eigenschaften eine zufriedenstellende Wirkung gegen adulte und juvenile Fasciola species in Schafen und Rindern bei gleichzeitig niedriger Toxizität aufweisen.

Es wurde nun gefunden, dass die in den erfindungsgemässen Mitteln enthaltenen Wirkstoffkombinationen, bestehend aus einer Verbindung der Formel I und der Verbindung der Formel II, eine hohe anthelminthische Wirksamkeit entfalten, die sich nicht nur mit breitem Wirkungsspektrum gegen Nematoden und Trematoden richtet, sondern eine stark ausgeprägte Wirkung gegen Fasciola hepatica in Schafen besitzt. Dabei ist als besonders vorteilhaft hervorzuheben, dass die den erfindungsgemässen Mitteln zugrundeliegenden Wirkstoffkombinationen bereits bei geringen Dosen totale Wirkung gegen Leberegel im Schaf erzielen, so dass Nebenwirkungen, die eine Beeinträchtigung der Entwicklung und der Nutzleistung der behandelten Tiere während der Behandlungszeit verursachen könnten, vermieden werden. Die gegen Fasciola hepatica

gerichtete Wirkung der erfindungsgemässen Wirkstoffkombinationen liegt signifikant über der additiven Wirkung der die Kombinationen bildenden Einzelverbindungen. Darüber hinaus ist das durch die erfindungsspezifische Zusammensetzung bedingte breite Wirkungsspektrum von besonderem Vorteil. So können die an Helminthiasis erkrankten Tiere mit einer einzigen Verabreichung der erfindungsgemässen Wirkstoffkombinationen gleichzeitig sowohl gegen Nematoden als auch gegen Trematoden aller Entwicklungsstadien behandelt werden. Es werden vor allem auch solche Nematoden-Stämme erfasst, die gegen Thiabendazol (2-[4-Thiazolyl]-benzimidazol) sowie teilweise auch gegen andere Benzimidazolderivate Resistenzerscheinungen aufweisen.

Als bevorzugt ist folgende Kombination anzusehen:

A) 5-Chlor-6-(2',3'-dichlorphenoxy)-2-methylthiobenzimidazol mit

B) L-(-)-2,3,5,6-Tetrahydro-6-phenylimidazo[2,1-b]-thiazol ("Levamisol").

Für die in den erfindungsgemässen Wirkstoffkombinationen massgebenden Gewichtsverhältnisse von Verbindungen der Formel I zur Verbindung der Formel II gilt der Bereich von 1 : 5 bis 5 : 1. Zur Entfaltung der synergistischen Wirkung ist das Kombinationsverhältnis von 1 : 5 bis 1 : 1 als besonders günstig anzusehen. Die einzusetzende Wirkstoffmenge der Verbindung der Formel I liegt bevorzugt bei unter 6 mg AS/kg Körpergewicht.

Die anthelminthische Wirkung der erfindungsgemässen Wirkstoffkombinationen von Verbindungen der Formel I mit der Verbindung der Formel II wurde anhand des folgenden Versuches geprüft:

**Versuch an mit Fasciola hepatica und Nematoden infizierten Schafen**

Schafe wurden artifiziell mit Metacercarien von Fasciola hepatica infiziert. Zu diesem Zweck wurden die Metacercarien in einer wässrigen 0,4 %-igen Suspension von Carboxymethylcellulose direkt in den Rumen der Versuchstiere injiziert. Gleichzeitig wurden die Schafe oral mit je 10 000 bzw. 5 000 infektiösen Larven (L 3) von Benzimidazolresistenten Trichostrongylus colubriformis und Haemonchus contortus infiziert. Für die Medikierung wurden die Versuchstiere in gleichgrossen Gruppen und in eine Kontrollgruppe eingeteilt. Anschliessend wurden die Wirkstoffkombinationen, in denen der Anteil von Wirkstoff der Formel I 2 - 5 mg/kg und der Anteil Wirkstoff der Formel II 3 - 7 mg/kg betrugen, an die Versuchstiere verabreicht.

Während des Versuchs wurden die Schafe in Gehegeboxen unter Normalbedingungen gehalten.

Zur Prüfung der Wirkung der einzelnen Wirkstoffkombinationen wurden gemäss Standardtechnik in bestimmten Zeitinvervallen nach Infizierung und Medikierung die mit dem Kot ausgeschiedenen Helmintheneier gezählt. Anschliessend wurden die Versuchstiere getötet und die Anzahl der vorhandenen adulten Helminthen festgestellt.

Die erhaltenen Resultate der medikierten und der Kontrollgruppen wurden fur die Auswertung verglichen.

Ergebnis: Die den erfindungsgemässen Mitteln zugrundeliegenden Wirkstoffkombinationen zeigen 95- bis 100 %-ige Wirksamkeit gegen Fasciola hepatica. Ihre Wirkung liegt bei vergleichbaren oder sogar niedrigeren Dosierungen signifikant über der additiven Wirkung der die Kombinationen bildenden Einzelverbindungen.

Bei den therapeutisch wirksamen Dosen der Wirkstoffkombinationen wurden an den Versuchstieren klinisch keine Toxizitätssymptome festgestellt. Die Tiere hatten ein gesundes Aussehen.

**Beispiel: Vergleichsversuchsergebnis**

Indikation: Fasciola hepatica

Anzahl Versuchstiere (Schafe): 19

**Tabelle**

| Schafe Nr. | Substanz bzw. Substanzgemisch (Dosis mg/kg) | EPG 12. Woche* | EPG 13. Woche* | adulte Helminthen Anzahl | adulte Helminthen Mittelwert | adulte Helminthen Reduktion in % |
|---|---|---|---|---|---|---|
| 1 | | 210 | 130 | 58 | | |
| 2 | I (2,5) | 160 | 160 | 65 | 48,5 | 52,7 |
| 3 | | 120 | 180 | 51 | | |
| 4 | | 20 | 15 | 20 | | |
| 5 | | 990 | 1140 | 108 | | |
| 6 | II (6,75) | 160 | 250 | 71 | 92,8 | 9,6 |
| 7 | | 226 | 250 | 89 | | |
| 8 | | 225 | 810 | 103 | | |
| 9 | | 0 | 0 | 3 | | |
| 10 | I + II (2,5 + 6,75) | 170 | 210 | 43 | 23,0 | 77,6 |
| 11 | | 0 | 50 | 19 | | |
| 12 | | 0 | 30 | 27 | | |
| 13 | | 710 | 680 | 99 | | |
| 14 | | 400 | 670 | 60 | | |
| 15 | unbehandelte | 1290 | 1270 | 113 | | |
| 16 | Kontroll- | 580 | 660 | 130 | 102,6 | |
| 17 | Tiere | 860 | 850 | 99 | | |
| 18 | | 570 | 410 | 110 | | |
| 19 | | 600 | 1450 | 116 | | |

**Legende:**

Substanz I: 5-Chlor-6-(2',3'-dichlorphenoxy)-2-methylthiobenzimidazol
Substanz II: L-(-)-2,3,5,6-Tetrahydro-6-phenylimidazo[2,1-b]-thiazol ("Levamisol")
EPG: Anzahl Eier pro gr Faeces

* nach Infizierung

Die erfindungsgemässen Wirkstoffkombinationen werden zur Bekämpfung parasitärer Helminthen bei Haus- und Nutztieren, wie Rindern, Schafen und Ziegen verwendet. Sie können den Tieren sowohl als Einzeldosis wie auch wiederholt verabreicht werden, wobei die einzelnen Gaben je nach Tierart zwischen 0,5 und 7,5 mg pro kg Körpergewicht betragen. Durch eine protrahierte Verabreichung erzielt man in manchen Fällen eine bessere Wirkung oder man kann mit geringeren Gesamtdosen auskommen. Die Wirkstoffe bzw. die sie enthaltenden Gemische können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen, Suspensionen (Drenchs), Pulver, Tabletten, Bolussen oder Kapseln peroral den Tieren verabreicht werden. Zur Bereitung dieser Applikationsformen dienen zum Beispiel übliche feste Trägerstoffe wie Kaolin, Talkum, Bentonit, Kochsalz, Calciumphosphat, Baumwollsaatmehl oder mit den Wirkstoffen nicht reagierende Flüssigkeiten wie Öle und andere, für den tierischen Organismus unschädliche Lösungs- und Verdünnungsmittel. Soweit die physikalischen und toxikologischen Eigenschaften von Lösungen oder Emulsionen dies zulassen, können die Wirkstoffkombinationen den Tieren auch beispielsweise subcutan injiziert oder mittels der Pour-on-Methode appliziert werden. Ferner ist eine Verabreichung der Wirkstoffe an die Tiere auch mittels Lecksteinen (Salz) oder Molasse-Blöcken möglich.

Liegen die anthelminthischen Mittel in Form von Futterkonzentrat vor, so dienen als Hauptträgerstoffe Futtermaterialien wie beispielsweise Heu, Leistungsfutter, Futtergetreide oder Proteinkonzentrate. Dem Futter

können ausser den Wirkstoffen noch Zusatzstoffe, Vitamine, Antibiotika, Chemotherapeutika oder andere Pestizide, vornehmlich Bakteriostatika, Fungistatika, Coccidiostatika oder auch Hormonpräparate, Stoffe mit anaboler Wirkung oder andere das Wachstum begünstigende, die Fleischqualität von Schlachttieren beeinflussende oder in anderer Weise für den Organismus nützliche Stoffe beigemischt sein. Auch können sie mit Insektiziden und Akariziden kombiniert werden, wodurch ihre Wirkung verbreitert und an gegebene Umstände angepasst wird.

Die Herstellung der erfindungsgemässen anthelminthischen Mittel erfolgt in an sich bekannter Weise durch inniges Vermischen und Vermahlen der Wirkstoffkombinationen, bestehend aus Verbindungen der Formel I und der Verbindung der Formel II mit geeigneten Trägerstoffen, gegebenfalls unter Zusatz von gegenüber den Wirkstoffen inerten Dispersions- oder Lösungsmitteln.

Die erfindungsgemässen anthelminthischen Mittel können beispielsweise in folgenden Zubereitungsformen angewendet werden:

**Feste Zubereitungsformen:**

Granulate, Umhüllungsgranulate, Imprägnierungsgranulate und Homogengranulate. In Wasser dispergierbare Wirkstoffkonzentrate (Wettable Powder)

**Flüssige Zubereitungsformen:**

Lösungen, Pasten, Emulsionen, insbesondere gebrauchsfertige Suspensionen (Drenches).

Die Korngrösse der Trägerstoffe beträgt für Stäubemittel und Spritzpulver zweckmässig bis ca. 0,1 mm und für Granulate 0,01 - 0,5 mm.

Die Konzentrat ion der Wirkstoffkombinationen in den festen Aufarbeitungsformen beträgt 0,5 bis 80 %, in den flüssigen Zubereitungsformen 0,5 bis 50 %.

Diesen Gemischen können ferner die Wirkstoffkombinationen stabilisierende Zusätze und/oder nicht ionische, anionaktive und kationaktive Stoffe zugegeben werden, die beispielweise eine bessere Benetzbarkeit (Netzmittel) sowie Dispergierbarkeit (Dispergatoren) gewährleisten.

**Beispiel**

**In Wasser dispergierbare Pulvermischung**

| | |
|---|---|
| 25 Gew.-Teile | Wirkstoffkombination von Verbindungen der Formel I mit der Verbindung der Formel II werden in einem Mischapparat mit |
| 7,5 Gew.-Teilen | eines aufsaugenden Trägermaterials beispielsweise Kieselsäure und |
| 59,4 Gew.-Teilen | eines Trägermaterials beispielsweise Bolus alba oder Kaolin und |
| 0,5 Gew.Teilen | Ölsäure und |
| 5,3 Gew.-Teilen | Octylphenylpolyglykoläther |
| 2,3 Gew.-Teilen | Stearylbenzimidazol-Derivate intensiv vermischt. |

Diese Mischung wird auf einer Stift- oder Luftstrahlmühle bis zu einer Partikelgrösse von 5 - 15 μm gemahlen. Das so erhaltene Spritzpulver gibt in Wasser eine gute Suspension.

**Patentansprüche**

1. Mittel zur Bekämpfung parasitärer Helminthen, dadurch gekennzeichnet, dass es als aktive Komponente eine Wirkstoffkombination bestehend aus mindestens einer Verbindung der Formel I

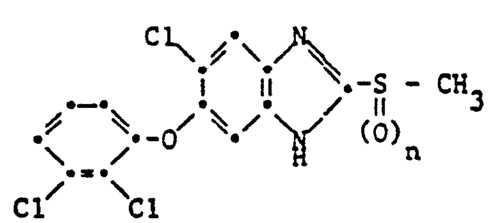

(I),

in welcher n gleich 0 oder 1 bedeutet, und der Verbindung der Formel II

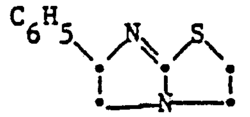

(II)

einschliesslich der unter die Formel II fallenden Isomeren sowie ihrer Säureadditionssalze in einer eine synergistische Wirkung erzeugenden Menge enthält zusammen mit geeigneten Trägerstoffen und/oder Verteilungs- und Verdünnungsmitteln.

2. Mittel zur Bekämpfung parasitärer Helminthen gemäss Anspruch 1, dadurch gekennzeichnet, dass es als aktive Komponente eine Wirkstoffkombination bestehend aus
5-Chlor-6-(2',3'-dichlorphenoxy)-2-methylthiobenzimidazol
und
L-(-)-2,3,5,6-Tetrahydro-6-phenylimidazo[2,1-b]-thiazol
zusammen mit geeigneten Trägerstoffen und/oder Verteilungs- und Verdünnungsmitteln enthält.

3. Mittel nach den Ansprüchen 1 und 2, dadurch gekennzeichnet, dass in der Wirkstoffkombination das Gewichtsverhältnis von Verbindungen der Formel I zur Verbindung der Formel II 1 : 5 bis 5 : 1 beträgt.

4. Mittel nach den Ansprüchen 1 und 2, dadurch gekennzeichnet, dass in der Wirkstoffkombination das Gewichtsverhältnis von Verbindungen der Formel I zur Verbindung der Formel II 1 : 5 bis 1 : 1 beträgt.

5. Mittel gemäss den Ansprüchen 1 - 4 zur Bekämpfung von Trematoden.

6. Mittel gemäss den Ansprüchen 1 - 4 zur Bekämpfung von Leberegeln (Fasciola hepatica).

7. Verwendung von Wirkstoffkombinationen gemäss den Ansprüchen 1 - 4 zur Herstellung eines anthelminthischen Mittels zur Anwendung in der Bekämpfung von parasitären Helminthen.

8. Verwendung von Wirkstoffkombinationen gemäss den Ansprüchen 1 - 4 zur Herstellung eines anthelminthischen Mittels zur Anwendung in der Bekämpfung von Trematoden.

9. Verwendung von Wirkstoffkombinationen gemäss den Ansprüchen 1 - 4 zur Herstellung eines anthelminthischen Mittels zur Anwendung in der Bekämpfung von Leberegeln (Fasciola hepatica).

10. Verwendung von Wirkstoffkombinationen gemäss den Ansprüchen 1 - 4 zur Herstellung eines anthelminthischen Mittels zur Anwendung in der Bekämpfung von Helminthen, wobei der Mengenanteil der Verbindung der Formel I bei weniger als 6 mg AS/kg Körpergewicht liegt.

## Claims

1. A composition for controlling parasitic helminths, which contains, as active component, a combination comprising at least one compound of formula I

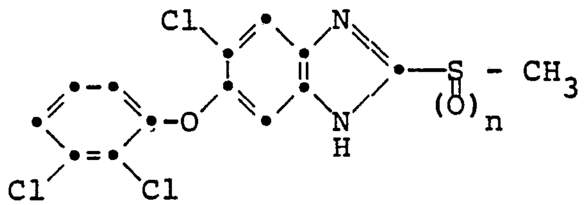

(I),

wherein n is 0 or 1, and the compound of formula II

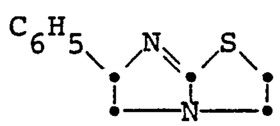

(II)

including the isomers thereof and the acid addition salts thereof, in an amount sufficient to produce a synergistic effect, together with suitable carriers and/or dispersants and diluents.

2. A composition for controlling parasitic helminths according to claim 1, which contains, as active component, a combination comprising
5-chloro-6-(2',3'-dichlorophenoxy)-2-methylthiobenzimidazole and
L-(-)-2,3,5,6-tetrahydro-6-phenylimidazo[2,1-b]-thiazole,
together with suitable carriers and/or dispersants and diluents.

3. A composition according to either claim 1 or claim 2, wherein the weight ratio of compounds of formula I to the compound of formula II in the combination is 1 : 5 to 5 : 1.

4. A composition according to either claim 1 or claim 2, wherein the weight ratio of compounds of formula I to the compound of formula II in the combination is 1 : 5 to 1 : 1.

5. A composition according to any one of claims 1 to 4 for controlling trematodes.

6. A composition according to any one of claims 1 to 4 for controlling liver flukes (Fasciola hepatica).

7. Use of a combination as claimed in any one of claims 1 to 4 for the preparation of an anthelmintic composition for application in the control of parasitic helminths.

8. Use of a combination as claimed in any one of claims 1 to 4 for the preparation of an anthelmintic composition for application in the control of trematodes.

9. Use of a combination according to any one of claims 1 to 4 for the preparation of an anthelmintic composition for application in the control of liver flukes (Fasciola hepatica).

10. Use of a combination according to any one of claims 1 to 4 for the preparation of an anthelmintic composition for application in the control of helminths, in which the amount of compound of formula I is less than 6 mg of active ingredient per kilogram of body weight.

**Revendications**

1. Moyen pour combattre les helminthes parasites, caractérisé en ce qu'il comporte comme composants actifs une combinaison d'agents actifs constituée par au moins un composé de formule I

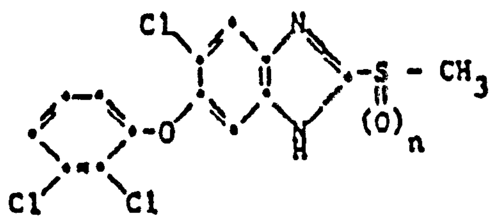

(I),

dans lequel n est égal à 0 ou 1, et le composé de formule II

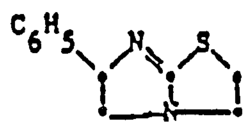

(II)

y compris les isomères conformes à la formule II ainsi que leurs sels d'addition acide, dans une quantité produisant une action synergétique, avec des supports et/ou des agents de dispersion et des diluants adaptés.

2. Moyen pour combattre les helminthes parasites conforme à la revendication 1, caractérisé en ce qu'il comporte comme composants actifs une combinaison d'agents actifs constituée par
le 5-chloro-6-(2',3-dichlorophénoxy)-2-méthylthiobenzimidazole
et
le L(-)-2,3,5,6-tétrahydro-6-phénylimidazo[2,1-b]-thiazole
avec des supports et/ou des agents de dispersion et des diluants adaptés.

3. Moyen selon l'une quelconque des revendications 1 ou 2, caractérisé en ce que, dans la combinaison d'agents actifs, le rapport en poids des composés de formule I par rapport au composé de formule II varie de 1: 5 à 5 : 1.

4. Moyen selon l'une quelconque des revendications 1 ou 2, caractérisé en ce que, dans la combinaison des agents actifs, le rapport en poids des composés de formule I par rapport au composé de formule II varie de 1 : 5 à 1 : 1.

5. Moyen selon l'une quelconque des revendications 1 à 4 pour combattre les trématodes.

6. Moyen selon l'une quelconque des revendications 1 à 4 pour combattre la douve du foie (fasciola hepatica).

7. Utilisation de combinaisons d'agents actifs selon l'une quelconque des revendications 1 à 4 pour réaliser un moyen anthelmintique pour emploi dans la lutte contre les helminthes parasites.

8. Utilisation de combinaisons d'agents actifs selon l'une quelconque des revendications 1 à 4 pour réaliser un moyen anthelmintique pour emploi dans la lutte contre les trématodes.

9. Utilisation de combinaisons d'agents actifs selon l'une quelconque des revendications 1 à 4 pour réaliser un moyen anthelmintique pour emploi dans la lutte contre la douve du foie (fasciola hepatica).

10. Utilisation de combinaisons d'agents actifs selon l'une quelconque des revendications 1 à 4 pour réaliser un moyen anthelmintique pour emploi dans la lutte contre les helminthes, dans lequel la quantité du composé de formule I est inférieure à 6 mg AS/Kg du poids du corps.